Europäisches Patentamt

European Patent Office

Office européen des brevets

(19)

(11) EP 1 191 103 A1

(12) EUROPEAN PATENT APPLICATION

(43) Date of publication:
27.03.2002 Bulletin 2002/13

(51) Int Cl.$^7$: C12N 15/82, C12N 9/10, C12N 15/31, A01H 1/00

(21) Application number: 00610096.0

(22) Date of filing: 21.09.2000

(84) Designated Contracting States:
AT BE CH CY DE DK ES FI FR GB GR IE IT LI LU MC NL PT SE
Designated Extension States:
AL LT LV MK RO SI

(71) Applicant: DLF-Trifolium
4660 Store Heddinge (DK)

(72) Inventors:
• Mikkelsen, Lisbeth
  4673 Roedvig Stevns (DK)

• Roulund, Niels
  4660 Store Heddinge (DK)
• Luebeck, Mette
  Arnakke, 4390 Vipperoed (DK)
• Luebeck, Peter
  Arnakke, 4390 Vipperoed (DK)
• Okkels, Finn Thyge
  4000 Roskilde (DK)
• Jensen, Dan Funck
  1903 Frederiksberg C (DK)

(54) **Grass containing genetically modified endophytes**

(57)    The present invention relates to grass that is infected with a genetically modified fungal endophyte. The endophyte has been genetically transformed with a gene and produces elevated levels of a plant growth regulator. When grass is infected with the transformed endophyte it becomes exposed to the growth regulators and responds by altered morphological characters. Since the endophyte is seed-borne, the novel phenotype can be maintained and propagated by allowing the endophyte infected plant to set seed. The seeds can then be used for agricultural or related purposes.

## Figure 6

EP 1 191 103 A1

**Description**

**FIELD OF INVENTION**

The present invention relates to a method for altering the characteristics of a plant.

**BACKGROUND OF THE INVENTION**

Endophytes are symbiotic fungi that naturally inhabit plants. Various species of the genus *Neotyphodium* infect grasses and endophyte infected perennial ryegrass is widespread in Australia and New Zealand and has been found throughout Europe (Latch et al., 1987). It is well documented that these fungal endophytes confer resistance to the grasses against herbivory insects, eg Argentine stem weevil (Hartley *et al.,* 1982), sod webworm (Funk *et al.,* 1983) and aphids (Clement *et al.,* 1991). The endophyte/grass interaction produces various alkaloids, and peramine (Rowan & Gaynor, 1986) and several not yet identified alkaloids (Ball *et al.,* 1994) are believed to be responsible for the deterrence or reduced growth of insects.

Researchers have documented that endophyte infected tall fescue is more tolerant to drought stress and pathogens, but the same has not been found for endophyte infected perennial ryegrass (Barker et al., 1997; Latch, 1997). Some studies suggest that endophytes may alter the morphological characters of the host plant, such as biomass production, tillering and seed germination, but the results are confusing ( Latch, Hunt & Musgrave, 1985; Clay, 1987; Clay et al., 1993; Cheplick, 1997). The mechanisms of the observed effects of the endophytes on the host plants are not known.

Cytokinins are a group of phytohormones known to affect plant growth. Cytokinins were discovered in the 1950s and identified as factors promoting cell division in plants (Miller *et al.,* 1956). In addition to the ability of cytokinins to promote or induce cell division, they are known to interact with other phytohormones for *de novo* bud formation, release of buds from apical dominance, leaf expansion, reproductive development, senescence of plants and regulation of seed germination (Mok, 1994).

The cloning of isopentenyl transferase encoding genes from *Agrobacterium tumefaciens* (*tmr*, *ipt, tzs)* opened for the possibilities for studying endogenous produced cytokinins and the effects on plant growth. Exogenous application of cytokinin, cytokinin producing transgenic plants or mutants defective in cytokinin metabolism have been important steps towards understanding cytokinin biosynthesis and mode of action in plants. The gene encoding an isopentenyl transferase has not yet been identified in plants, despite several attempts (Chen, 1982; Reinecke et al., 1992). Cytokinin secreting genes have been isolated from various bacteria, as *Agrobacterium tumefaciens, A. rhizogenes, Pseudomonas solanacearum* and *P. syringae* pv. *savastanoi* (Akiyoshi, Regier & Gordon, 1987) and *Rhodococcus fascians* (Crespi *et al.,* 1992). All these genes code for the isopentenyl transferase enzyme (IPT).

The *tzs* gene was cloned from *A. tumefaciens* and shown to encode the enzyme dimethylallyl transferase which is equivalent to isopentenyl transferase (Beaty *et al.,* 1986). The gene was reported to be responsible for the secretion of primarily transzeatin, but also isopentenyl adenine, isopentenyl adenosine and transzeatin riboside. These are active cytokinins together with dihydrozeatin and dihydrozeatin riboside among others.

The application of cytokinin and other plant growth regulators to growing plants could be desirable in for example agriculture. In the case of cytokinins, such an approach would allow the plant to benefit from the growth promoting and senescens delaying effects of the growth regulator.

An alternative way to administer plant growth regulators could be by genetic transformation of the plant. Genetic transformation of plants with genes involved in the metabolism of cytokinin can be used to alter the growth and other characteristics of the plant. This has been demonstrated in a number of cases and in general the physiological responses associated with *ipt* gene expression in plants correlate with those frequently observed following exogenous application of cytokinins. However, transformation of plants using the *ipt* gene often results in degenerated plants, which are stunted with no developing roots, depending on the level of gene expression and cytokinin accumulation in the plants (Li, Hagen & Guilfoyle, 1992).

Inducible promoters have been used to prevent the detrimental accumulation of the cytokinins, and the most widely used are the heat shock promoters isolated from various organisms. Fusion of the *ipt* gene to inducible promoters with a low level of constitutive expression often results in visible phenotypic changes in the absence of gene induction. The *ipt* gene placed under the control of a heat-inducible promoter resulted in an overproduction of cytokinin even without heat shock induction of the transgenic plants. Shorter plants with larger side shoots and delayed senescence of transgenic tobacco plants (Smart *et al.*, 1991) and *Nicotiana plumbaginifolia* (Smigocki, 1991), and a reduced root system, increased height and branching, delayed flowering, and increased seed yield of transgenic *Brassica napus* (Roeckel, Oancia & Drevet, 1998) was observed without heat-shock induction of the plants (Medford *et al.,* 1989). Induction of the *ipt* gene did not change the phenotypic appearance any further, even though cytokinin concentration increased dramatically (20-200-fold), suggesting that the cytokinin response may be saturated at relatively modest increases in cytokinin level (Hobbie, Timpte & Estelle, 1994).

Other approaches have been used to direct the induction of cytokinin expression. Promoters inducible by auxin (Li, Hagen & Guilfoyle, 1992), wounding (Smigocki, 1995), light treatment (Garnier *et al.*, 1996), tetracycline (Faiss *et al.*, 1997), copper (McKenzie *et al.*, 1998) or senescens (patent application US5689042) have been used in front of the *ipt* gene. However, all the above-mentioned inducible promoters were reported to cause a low level of constitutive expression except the copper inducible promoter. Tissue specific promoters have also been used for direction of cytokinin production to specific tissues or developmental stages of the plant. An ovaria specific promoter, caused an increase of cytokinin in ovaries of transgenic tomato plants (Martineau *et al.*, 1995).

Despite the succesfull expression of cytokinins in plants the agricultural application of this approach is, however, complicated by at least two factors. Firstly, it has proven difficult to restrict the production of cytokinin to certain tissues and secondly, many important crops are recalcitrant to genetic transformation and even if this is possible, it requires a very long time to do so. More over, if transgenic plants are released to the environment there is a potential risk that the transgene will unintendedly spread to similar or related species through pollen. This is of particular concern if the crop of interest is a grass, which is a wind pollinator and typically is native to the wild flora.

## DETAILED DESCRIPTION OF THE INVENTION

We have used a different approach for directing *ipt* gene expression and cytokinin production into perennial ryegrass. The presence of the *Neotyphodium lolii* endophyte in leaf sheaths, stems and meristems of perennial ryegrass may serve as an excellent organism for delivering cytokinins into the host plant. The observed severe effects of the *ipt* gene expression in transgenic plants, as stunting and limitation of root growth may be prevented, by the use of *ipt* expressing *N. lolii,* since the endophyte is not located in the roots (Herd *et al.,* 1997). Furthermore, *N. lolii* is obligate symbionts that apparently do not escape the plant and are only transmitted through seeds. We have used this approach to affect the growth of perennial ryegrass by infection with a transgenic *N. lolii* endophyte expressing the *tzs* gene.

The fungal endophyte *Neotyphodium* has previously been transformed and used for infection of perennial ryegrass (Murray et al., 1992) or tall fescue (Tsai et al., 1992). In these experiments exclusively marker genes were transferred to the endophyte and the grasses did not accquire any altered morphological phenotype. Murray et al. (1992) suggested that endophytes could be used 'as a vehicle for introducing foreing genes into grasses' and demonstrated that the *uidA* gene was expressed in the endophyte after infection of perennial ryegrass. They did, however, not suggest what other kind of genes could be used. Recently, Yunus et al. (1999) have reported the transformation of *N. lolii* with the *iaaM* gene. This gene enabled the endophyte to produce the plant growth regulator indole-3-acetic acid and the intermediate indole-3-acetamide, which belong to the group of auxins. The authors suggested that these endophytes could be used for infection of grasses but they did not demonstrate this.

### Materials and methods

#### Strains and isolates

*Escherichia coli* strain MC1061 was used as host for plasmid amplification and cloning (Sambrook, Fritsch & Maniatis, 1987). The endophyte isolates LM37-5, LM40-5, and LM41-4 were isolated from leaf sheaths of *L. perenne* L. var. Apollo, Laredo, and breeding line Lp26, respectively, as previously described (Latch, Christensen & Samuels, 1984). The isolates emerged from the leaf sheaths after 3-4 weeks, and the isolates were slow growing and had the appearance of *N. lolii* (Christensen, Latch & Tapper, 1991). The endophyte isolates were identified as *N. lolii* with the universal primed (UP) PCR fingerprinting technique using the reference *N. lolii* isolates Lp5 (Shaw *et al.,* 1997) and CBS232.84 (Centraalbureau voor Schimmelcultures, The Netherlands) (Latch, Christensen & Samuels, 1984). Two related isolates were used for comparison: Lp1 (Christensen *et al.,* 1993), a *Neotyphodium* hybrid (LpTG-2) from *L. perenne* and E8, an *Epichloë typhina* from *L. perenne.* The endophyte mycelium was cultured on 1.5 % (w/v) potato dextrose agar (PDA; Difco Laboratories, Detroit, Mich.), pH 6.5, at 21°C in the dark. Cultures were kept in the host plant or on agar plugs in 15 % glycerol at -70°C.

#### Plasmids

Plasmid pAN7-1 (Punt et *al.,* 1987) contains the *hph* (hygromycin B phosphotransferase) and pNOM102 (Roberts *et al.,* 1989) contains the *uidA* (β-glucuronidase). Both genes are from *Escherichia coli*, and both are coupled to the *Aspergillus nidulans gpd* promoter and the *A. nidulans trpC* terminator. Two constructs were developed for inserting the *tzs* gene (Beaty et *al.,* 1986) encoding dimethylallyl pyrophosphate: 5'-AMP dimethylallyl transferase (DMA transferase) (Chen & Melitz, 1979) behind a fungal promoter. The pNOM102 plasmid was used as a vector for insertion of the *tzs* gene. The β-glucuronidase gene was removed with *Bam*HI and *Nco*I restriction enzymes, and the remaining 5.7Kbp fragment was ligated to the PCR amplified *tzs* fragment.

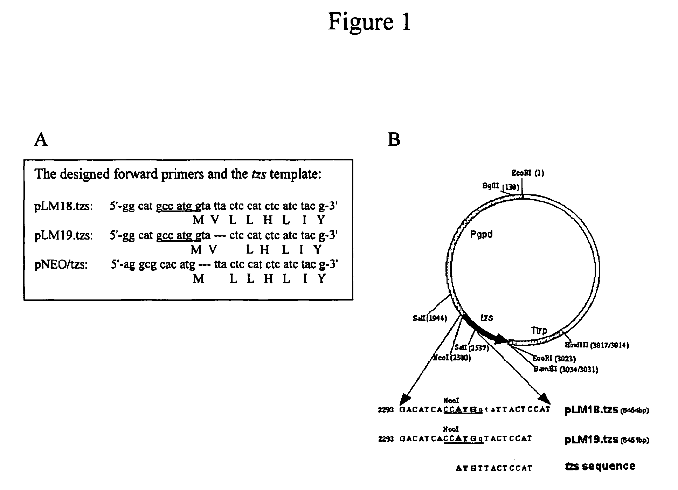

The pNEO/tzs plasmid (F. Okkels, DLF-Trifolium) was used as template for PCR amplification of the *tzs* gene. The pNEO/tzs contained the *tzs* gene from the TI-plasmid of *Agrobacterium tumefaciens* (Beaty *et al.*, 1986) inserted at the *Bgl*II site in the pNEO plasmid (Colbere-Garapin *et al.*, 1981). In order to generate a *Nco*I restriction site in the start codon region of the *tzs* gene it was necessary to alter the *tzs* sequence. Two strategies were followed. In pLM18.tzs the amino acid, valin was added between the 1st and the original 2nd amino acid. In pLM19.tzs the 2nd amino acid, leucin was substituted with valin. Two forward primers were designed which added a *Nco*I restriction site at the startcodon resulting in two slightly altered versions of the *tzs* gene as indicated in figure 1. The shared reverse primer added a *Bam*HI site after the stopcodon. The 29bp primer containing the last 20 bp of the tzs gene including stop codon and a BamHI site (underlined): 5'-cgc gga tcc tca ccg aat tcg cgt cag cg-3'.

The PCR amplification of the tzs sequences was carried out using Vent polymerase (New England Biolabs). The amplification conditions using the MiniCycler™, Model PTC-150 (MJ Research, Watertown, MA) were as follows: 25 cycles with DNA denaturation at 91 °C for 1 min (first denaturation step at 94 °C for 2 min), primer annealing at 60 °C for 1 min, and primer extension at 72 °C for 90 s (final extension step at 72 °C for 3 min).

The *tzs* region of the constructs was sequenced (DNA Technology A/S, Aarhus, Denmark) to verify that no mutations had occurred during the PCR amplification. Plasmid purification was performed using Qiagen midi prep kit (Qiagen Inc., Chatsworth, CA.) according to the manufacturers instructions.

Preparation of protoplasts and transformation

Preparation of protoplasts was done as previously described (Murray, Latch & Scott, 1992). The transformation procedure described by (Murray, Latch & Scott, 1992) was followed, with minor modifications. Spermidine was excluded because the batch obtained killed the protoplasts. The 40 % polyethylene glycol (PEG) solution was added in aliquots of 100, 200, 200, and, 400 µl, a total of 900 µl. The solution was gently mixed between each PEG addition. Aliquots of 500 µl of the transformation solution were added to 5 ml of 0.8% molten agar (50 °C) in complete medium (CM) (Oliver et *al.,* 1987), poured on CM agar plates and incubated at 21 °C. Hygromycin B was added the next day in 5 ml of 0.8 % molten CM agar to a final concentration of 200 mg l[-1] hygromycin B. Mycelium growth from regenerating protoplasts was observed in the stereo microscope after 8-10 days of incubation. Molten PD agar + 200 mg l[-1] hygromycin B was added 5 weeks after transformation. Putative transformed colonies were transferred to PDA containing 200 mg l[-1] hygromycin B about 6 weeks after transformation. Three transformation experiments were carried out, one with each of three isolates. Each transformation experiment was carried out as co-transformation using 4 µg of each of circular pAN7-1 + either pLM18.tzs or pLM19.tzs plasmid. Protoplasts regeneration was observed on nonselective medium.

Genomic DNA preparation and southern hybridization

Large-scale genomic DNA preparation was done using the procedure described by Moon *et al.* (1999) (Moon, Tapper & Scott, 1999). Southern blot was carried out by the method of Southern (Southern, 1975). The genomic DNA (2 µg) was digested with either *Bgl*II or *Cla*I restriction enzymes to determine the copy number and sites of integration. The plasmid pLM18.tzs and pLM19.tzs contain a single *Bgl*II restriction (fig. 1) and *Cla*I does not digest the plasmid. The *tzs* gene sequence was used as hybridisation probe. The 486 bp *tzs* fragment of the *Eco*RI + *Sal*I digested pNEO/tzs plasmid was isolated and labelled with [P[32]]-dCTP using the RediPrime™ II kit (Amersham Pharmacia Biotech, UK) according to the manufacturers instructions.

PCR of putative transformants

The forward and reverse primer recognized the *gpd* promoter and the *trpC* terminator, respectively, and amplified the gene sequence in between resulting in a 920bp fragment of the tzs plasmids or a 1.221bp fragment of the pAN7-1.

Sequence of forward primer was 5'-CTCCATCCTTCCCATCCCTTATTCC-3' and reverse primer was 5'-CTCCGGAGCTGACATCGACACCAAC-3'. The PCR reaction was carried out in 20 µl volume containing 10 mM Tris-HCl, pH 8.8, 3.5 mM MgCl$_2$, 50 mM KCl, 0.1 % Triton X-100, 0.2 mM dNTPs, 25 pmol of each primer, 1µl and 1µl of a 1:10 dilution of total genomic DNA, and 1 unit of DynaZyme version 2 polymerase (Finnzymes OY, Helsinki, Finland). The PCR conditions were the same as mentioned before in the plasmid section. Two µl PCR products (1/10 of the total reaction) were electrophoresed in 1.7 % 7 cm long agarose gels at 300 V with TBE buffer for 40 min. The gels were stained with ethidium bromide, and scanned using the Image-Master VDS (Pharmacia Biotech, Uppsala, Sweden).

Fungal growth conditions for cytokinin analysis

Mycelium was grown in 25 ml PD broth (PDB) in a 125 ml conical flask. Inoculum was prepared as follows. A square piece of mycelium (5x5mm) was excised from a 3-5 week old PDA plate culture. All excess agar was removed from the mycelium square. The mycelium was transferred to a 1.5 ml tube and macerated with a small pestle. The macerated mycelium was suspended in 0.5 ml PDB and transferred to the flask using a cut 1 ml disposable pipette tip. The cultures were incubated on a rotary shaker, 200 rpm, at 21 °C for 18 days. The culture filtrate was separated from the mycelium by filtration through a nylon filter. The culture filtrate was stored at -20 °C until subjected to analysis. The mycelium was freeze-dried for dry weight determination and DNA extraction. Two replicate flasks per isolate.

Cytokinin analysis - enzyme linked immunosorbent assay (ELISA).

The assay was performed in 96-well microtiter plates (Nunc, Roskilde, Denmark) as described by Ulvskov *et al.* (1992). [9R]Z-alkaline tracer K23/x and antiserum G31 were used for quantification of the zeatin group of cytokinins. [9R]iP-alkaline tracer K30/3 and antiserum G26 were used for quantification of the isopentenyl adenine group of cytokinins. Standards of respectively transzeatin and isopentenyl adenosine were included in each titer plate. Filtrates were centrifuged 10 min at 15.000 rpm in a microcentrifuge. Each filtrate was measured in quadruplicate.

Statistical analysis

Non-linear regression of the ELISA data using the four-parameter logistic equation was done using the software Prism ver. 2.01:

$$y = C + \frac{D - C}{1+(x/ED_{50})^{b}},$$

y =       absorbance of x
x =       concentration of transzeatin/isopentenyl adenosine
C =       bottom plateau
D =       top plateau
b =       hill slope
ED50 =       concentration that gives response halfway between bottom and top.

For all experiments $R^2 > 0.99$. SAS ver. 6.12 was used for statistical analysis.

Cytokinin analysis - high performance liquid chromatography (HPLC)

Preparative reverse-phase HPLC was performed as described by Ulvskov *et al.* (1992). The equipment consisted of liquid chromatograph LC-10AT, auto injector SIL-10AXL, system controller SCL-10AVP and a diode array detector SPD-M10AVP (Shimadzu Corporation), and a degasser ERC-3415$\alpha$ (ERC Inc.). Software: CLASS-VP release 5.02. Detection wavelength was set to 273 nm.
Solvent A: 90 % $CH_3OH$. Solvent B: 10 mM $CH_3COOH$. Column: Supelcosil LC-ABZ, 25 cm x 4.6 mm, 5 $\mu$m. Start conditions were 0 % A- 100 % B in 2 min, linear gradient to 10 % A- 90 % B at 5 min followed by a linear gradient to 75 % A - 25 % B at 30 min, and finally a linear gradient to 100 % A at 40 min, flow rate = 1.25 ml min$^{-1}$.
A C18 SepPak column (Waters Associates, Milford, MA) was prewashed with 20 ml methanol, 10 ml $H_2O$ and 10 ml 10 mM potassium phosphate buffer saline (PBS), pH 6.0. Culture filtrates of transformants were centrifuged 10 min at 8000 x g at 4 °C .The supernatant was diluted (1:1) with 10 mM potassium phosphate buffer, pH 6.0, and loaded onto the column, and washed with 10 ml 10 mM potassium phosphate buffer, pH 6.0, 5 ml $H_2O$. Cytokinins were eluted backwards from the column in 5 ml 80 % methanol. The methanol was evaporated in a vacuum centrifuge. The remaining solution was filtered through a 0.45 $\mu$m filter and loaded onto the HPLC column. Fractions of 1.25 ml were collected from 5 to 30 min. The methanol was evaporated in a vacuum centrifuge and PBS, pH 7.0, was added to a final volume of 2.50 ml for ELISA analysis.

Infection of plants and observation of plant growth

Seedlings of perennial ryegrass *L. perenne* var. Tivoli were inoculated with fungal mycelium as described by Latch and Christensen (Latch & Christensen, 1985). Infected plants were grown in the greenhouse. Vernalization was done in the cold house for 3 month. Two month after transfer to the warm house plants were observed for total number of shoots, heading date, number of flowering stems and plant height at flowering.

PCR of endophyte infected host plants

A single shoot from each plant, 3 cm of the basal part, was DNA extracted by the method of (Edwards, Johnstone & Thompson, 1991) except 400 μl of 5M potassium acetate was added 5 min after addition of extraction buffer and incubated on ice for additional 30 min. The DNA was subjected to PCR for verification of endophyte genomic DNA using the endophyte specific primers B10.1 and B10.2 (Moon, Tapper & Scott, 1999) amplifying a 178 bp fragment. Specific tzs primers were developed, and forward primer was: 5'-CGGGTCGATTTCGCTTCTCAA-3' and reverse primer: 5'-GCTGTCCTGCGCCATCTTCTG-3' amplifying a 407 bp fragment. All four primers (0.2 μM each) were mixed in a single PCR reaction together with 1.5 mM MgCl$_2$, 0.1 mM of each dNTP and 0.25 U Taq polymerase in 20 μl volume. PCR conditions as described by (Moon, Tapper & Scott, 1999).

**Examples**

Transformation

Cotransformation was carried out using three different isolates of *N. lolii.* DNA from the 13 resulting hygromycin B resistant transformants was PCR amplified to check for the tzs insert. The 7 pLM18.tzs transformants and the 4 of the 6 pLM19.tzs transformants amplified a fragment of 920 bp, indicating that the tzs gene was inserted into 11 of 13 transformants resulting in a cotransformation rate of 84 %. The transformation efficiency was 4 x 10$^{-6}$ of the original number of protoplasts, and 1 transformant μg$^{-1}$ DNA was obtained. The colony appearance of the transformants was similar to the original wild type isolate except a single very slow growing transformant.

Southern hybridisation of cytokinin transformants

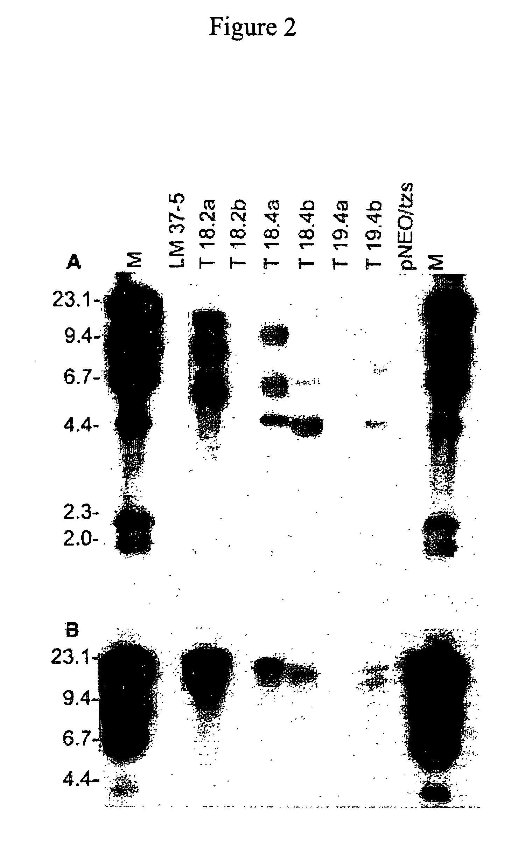

Five pLM18.tzs transformants and a single pLM19.tzs transformant were chosen for inoculation into perennial ryegrass seedlings, because of their cytokinin production (not shown). Each transformant was re-isolated from a single plant for comparison of the genomic DNA to the original transformant. Three of the transformants had kept the *tzs* inserts during growth in the host plant as shown in figure 2. No hybridization was observed of the *tzs* probe to the wild type DNA of LM37-5 indicating that the endophyte does not contain homologous sequences of the *tzs* gene.
The *Cla*I digest (figure 2B) showed hybridising fragments in the high molecular weight DNA region larger than 9 kb indicating that the tzs plasmid were inserted into the genomic DNA. The transformants T18.2, T18.4 and T19.4 had three or more inserts of the *tzs* gene before inoculation into plants. The T19.4 seemed to have a stable hybridization pattern having three tzs inserts both before infection and after re-isolation from the host plant. After re-isolation from the host plant the hybridization pattern of the transformants T18.2 and T18.4 had changed. T18.2 had 5 very strong hybridising fragments of which 3 seemed lost after re-isolation from the plant and the 2 remaining fragments were very faint as if they had been diluted indicating that T18.2 may be a chimaere originating from a heterokaryon protoplast. The stability of the transformants is further discussed below. The T18.4 had at least 3 hybridising fragments before inoculation to the host plant. After re-isolation, the transformant had lost one of the fragments suggesting that the transformant had excised a previously inserted gene. The *Cla*I digest showed 2 hybridising fragments of T 18.4 before and after re-isolation indicating that the genes were inserted at 2 different sites in the genome.

Cytokinin production of tzs transformants grown in liquid culture

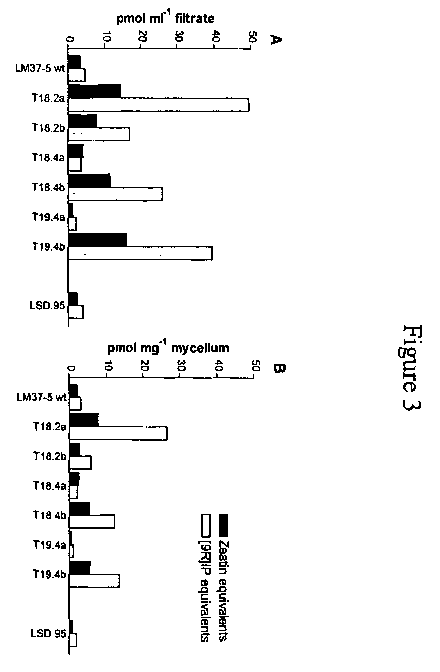

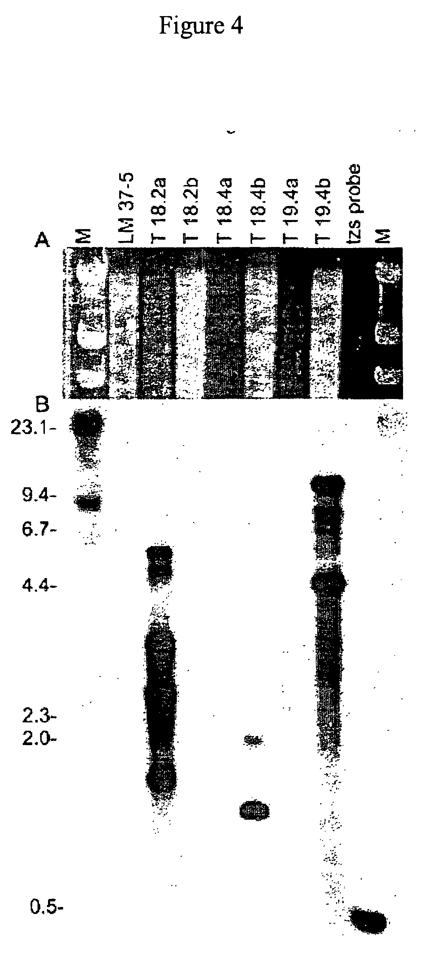

The transformants produced cytokinins in liquid potato dextrose broth (PDB) at varying levels (figure 3) as determined by immunoassay. The transformants produced both zeatin and isopentenyl adenine type cytokinins, and the level of isopentenyl adenine cytokinins was generally more than 2-fold higher than zeatin cytokinins.
The T18.2a and b, T18.4b and T19.4b produced relatively high levels of cytokinin significant different from the wild type isolate indicating that the *tzs* gene was expressed in the transformants and that the resulting dimethylallyl transferase produced the cytokinin secondary metabolites.
The southern hybridization of the DNA extracted from the mycelium from the liquid culture experiment verified the presence of the tzs gene in the mycelium of the transformants that produced cytokinins (figure 4). The hybridization pattern of the transformants seemed however to have changed between the two southern blots in figure 2 and 4 as will be discussed further.
The cross reactivity of the antisera used in the ELISA assays to the different cytokinins are listed in table 1. The G26 antiserum used in this study has cross reactivity similar to G304 in table 1 (Ulvskov, unpublished). The zeatin group cytokinins as determined by the G31 antiserum in figure 3 may consist primarily of the active zeatin riboside and zeatin, but recognises also the inactive zeatin-N9-glucoside. The isopentenyl adenine group may consist primarily of the active isopentenyl adenosine and isopentenyl adenine. Identification and composition of the cytokinins were verified by reverse phase HPLC.

Table 1.

| Antibody specificity of the polyclonal antisera used for the ELISA analysis as characterised by cross reactivity. The ligand used for the antigen and for the phosphatase conjugate (tracer) is set to 100%. The ability of the test compounds to displace the tracer is expressed relative to the displacement efficiency of the ligand (from Ulvskov *et al.*, 1992). | | |
| --- | --- | --- |
| Compound | G31 anti-zeatin riboside | G304 anti-isopentenyl adenosine |
| Zeatin | 71 | 0.4 |
| Zeatin riboside | 100 | 2 |
| Zeatin-O-glucoside | <0.1 | <0.1 |
| Zeatin-N9-glucoside | 46 | 0.4 |
| Dihydrozeatin | 10 | 39 |
| Dihydrozeatin riboside | 4 | 100 |
| Dihydrozeatin-N9-glucoside | 1.3 | |
| Isopentenyl adenine | 0.5 | |
| Isopentenyl adenosine | 0.8 | |

Reverse phase HPLC separation of cytokinins

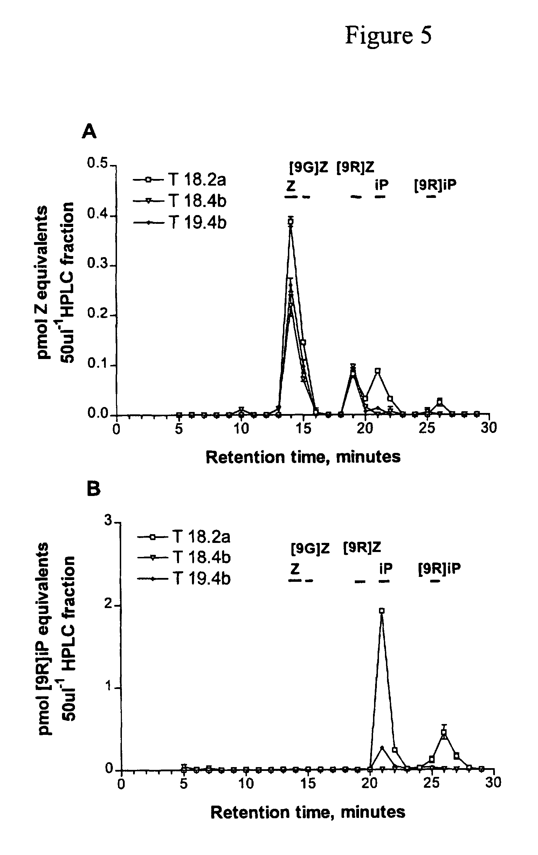

The culture filtrates from three of the highest producing isolates were subjected to reverse phase HPLC for identification and composition of the cytokinin metabolites produced by the transformants. The C15 column separated the zeatin and isopentenyl adenine, their corresponding riboside and the zeatin-N9-glucoside. Zeatin and dihydrozeatin could not be distinguished. Twenty-five fractions from each isolate were collected and the immunoreactive compounds were identified by ELISA (figure 5). The tzs transformants had produced both zeatin and isopentenyl adenine group cytokinins and their corresponding riboside. The major component of the zeatin group of cytokinins was zeatin, whereas a minor part was zeatin riboside. The peaks of isopentenyl adenine and isopentenyl adenosine (figure 4A) are due to cross reactivity of the G31 antiserum of less than 1 %. The isopentenyl adenine group of cytokinins seemed to consist primarily of isopentenyl adenine and a minor amount of the corresponding riboside isopentenyl adenosine. All three transformants showed similar peaks of the zeatin cytokinins, whereas only T18.2a and T19.4b had detectable levels of isopentenyl adenine cytokinins. The level of isopentenyl adenine cytokinins in the SepPak treated filtrate used for HPLC from the transformant T18.4b was also slightly reduced compared to T18.2a and T19.4b (data not shown) indicating that the isopentenyl adenine cytokinins recovered from the SepPak column may have been below detection level.

The major part of the endophyte transformant produced cytokinins seemed to be active compounds, although the formation of inactive cytokinins cannot be fully excluded, since the shoulder of the zeatin peak may consist partly of the inactive zeatin-N9-glucoside as the G31 cross reactivity is 65 % compared to zeatin. The antisera do not recognize the inactive O-glucosides.

It can be discussed if the wild type culture filtrate should have been included in the HPLC analysis, due to the low level of interfering compounds. We considered that the amount of the interfering compounds were too low to be detected after separation in the HPLC column without additional purification. However, the identification of the low level of interfering compounds from the wild type filtrate remains to be determined.

Effects on the host plant

Transformants were inoculated into the host plant (table 2). All the plants were infected with endophyte mycelium as tested with PCR using endophyte specific primers. All the plants infected with T19.4 contained mycelium with the tzs gene, whereas half of the T18.2 infected plants and all T18.4 infected plants apparently had lost the tzs gene in the mycelium.

Table 2.

| Perennial ryegrass var. Tivoli was infected with the tzs transformants. Infection was verified by PCR amplification of DNA isolated from the basal part of a single shoot from the plant using endophyte specific primers and tzs specific primers. | | |
|---|---|---|
| Transformant | Number of plants containing the transformed endophyte | Number of plants containing mycelium with the tzs gene |
| T 18.2 | 14 | 7 |
| T 18.4 | 5 | 0 |
| T 19.4 | 6 | 6 |

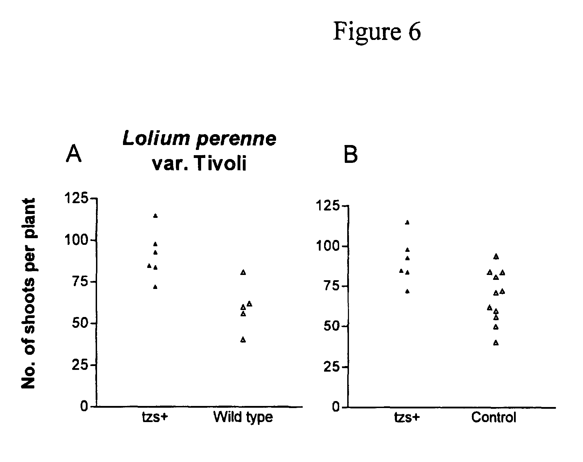

The total number of shoots, heading date, number of flowering shoots and plant height at flowering were noted. As the pLM18.tzs transformants seemed mitotically unstable, only plants containing the transformant T19.4 were useful for comparison to non-producers of cytokinin. The T19.4 is a transformant of the wild type isolate LM41-4. Plants infected with the wild type isolate LM41-4 (GUS transformants) were used as control group. Number of shoots were significant different at 5 % level. More shoots were observed on the plants infected with the T19.4 (figure 6). Heading date, number of flowering shoots and plant height at flowering were not significant different from the control group. The observed difference remained significant at 5% level when plants infected with the LM37-5 wild type were included in the control group. This was tested, due to the few control plants infected with LM41-4 wild type. It must be stated that the few plants of an open pollinated species may be too little material for making any firm conclusions, since the observed difference in this experiment by chance may be due to genotype differences. A more extensive experiment, including detailed observations of plant growth, needs to be carried out before any further conclusions can be made.

Stability of the transformants

The transformant T19.4 had a mitotically stable hybridization pattern during infection and re-isolation from the host plant.
The hybridization pattern was unchanged after re-isolation with three hybridising fragments indicating that the tzs gene was inserted in at least three copies. The sizes of the three fragments were similar in both southern hybridizations (figure 2 and 4), suggesting that the genomic DNA had not rearranged during subculture. The T19.4b re-isolated from plants produced a relatively high level of cytokinin. The transformed mycelium in each of the six infected plants contained the tzs gene verified by PCR. All the obtained results indicate that the T19.4 is a stable transformant as long as it is maintained in the plant. In contrast, the T19.4a (figure 4) seemed to have a changed hybridization pattern, yet the amount of DNA was poor a single faint hybridising fragment was observed. This fragment had rearranged to become shorter, and as the T19.4a did not produce cytokinin this rearrangement in the genome may have resulted in a non-functional gene. The T19.4a transformant had been sub cultured for more than a year on nonselective medium before it was used in the liquid culture experiment suggesting that the genomic DNA may rearrange during subculture. The hybridization pattern of mycelium DNA from each of the infected plants remains to be determined.
The transformants T18.2 and T18.4 seemed to be mitotically unstable as they both had lost hybridising fragments during infection and re-isolation from the host plant (figure 2). The heterogenous or unstable character of the transformed mycelium was also observed in the infected plants (table 2). All of the infected plants contained endophyte mycelium. However, most plants infected with the T18.2 and T18.4 transformants contained mycelium, which had lost the *tzs* gene, confirming that these transformants were mitotically unstable.
The transformant T18.2a, which had at least 5 hybridising fragments, produced a high level of cytokinins, whereas T18.2b with only 2 very faint fragments produced a lower level. The observed differences in number of *tzs* inserts may be a reason for the observed differences in cytokinin production. The T18.4a did not produce cytokinin suggesting that the *tzs* fragments (figure 2) may have been lost during subculture. This was also confirmed by PCR with tzs specific primers, as no *tzs* fragment was amplified (presence of genomic DNA was verified using general endophyte primers). The lack of hybridising fragments was not confirmed by the southern hybridization (figure 4), since the DNA extraction was very poor. Absence of hybridising fragments was due to the missing DNA.
The T18.4b produced a high level of cytokinin and presence of the *tzs* gene was confirmed in the southern hybridization, which revealed two hybridising fragments.

Both transformants, T18.2 and T18.4 had lost some of the *tzs* inserts during infection of the plant suggesting that the inserts were either excised from the genome or lost with a transformed nucleus in a heterokaryon originating from a multinucleate protoplast or a protoplast fusion product. The T18.2 may be a heterokaryon originating from a protoplast containing two transformed nuclei together with one or more wild type nuclei. The first transformed nucleus may have been lost during infection of plants, whereas the second transformed nucleus may have been lost in some of the mycelium used for the DNA extraction causing a dilution of the hybridising fragments.

Apart from excision of previously inserted fragments or loss of transformed nuclei, rearrangements in the genome seemed to have occurred during subculture on nonselective medium. The hybridising fragments of T18.2b were of similar size on both southern blots (about 7 and 9Kb), whereas the fragments of T18.2a had been rearranged resulting in shorter fragments. The shortest fragment (5.5 Kb) of T18.2a in figure 2A is of similar size as the longest fragment in figure 4B and may be an unchanged fragment. The other fragments were reduced from 6.3 - 15Kb in size to 1.6 - 5.1Kb.

The genomic DNA of the T18.4 had also rearranged, as the 2 fragments of 4.5 and 6.5 Kb in size shown in figure 2 was reduced to 1.3 and 2.1 Kb in size in figure 4. These rearrangements and reduction in fragment size had not affected the ability of producing cytokinins, although the level of cytokinin production may have been affected.

**REFERENCES**

Akiyoshi, D. E., Regier, D. A. & Gordon, M. P. (1987) Cytokinin production by *Agrobacterium* and *Pseudomonas* spp. *Journal of Bacteriology* **169:** 4242-4248.

Ball,O.J.P., Christensen,M.J., Prestidge,R.A., & Popay,A.J. (1994) Effect of selected isolates of *Acremonium* endophyte on adult black beetle (*Heteronychus arator*) feeding. New Zealand Plant Protection Society; Rotorua; New Zealand.

Barker, D. J., Hume, D. E. & Quigley, P. E. (1997) Negligible physiological responses to water deficit in endophyte-infected and uninfected perennial ryegrass.: p. 137-139. *Neotyphodium/grass interactions.* New York (USA). Plenum Press.

Beaty, J. S., Powell, G. K., Lica, L., Regier, D. A., MacDonald, E. M. S., Hommes, N. G. & Morris, R. O. (1986) Tzs, a nopaline Ti plasmid gene from *Agrobacterium tumefaciens* associated with trans-zeatin biosynthesis. *Molecular and General Genetics* **203:** 274-280.

Brzobohaty, B., Moore, I. & Palme, K. (1994) Cytokinin metabolism: implications for regulation of plant growth and development. *Plant Mol Biol.* **26:** 1483-1497.

Campos, N., Bako, L., Feldwisch, J., Schell, J. & Palme, K. (1992) A protein from maize labeled with azido-IAA has novel β-glucosidase activity. *Plant Journal* **2:** 675-684.

Chen, C. M. (1982) Cytokinin biosynthesis in cell-free systems. In *Plant Growth Substances* (P. F. Wareing, eds): pp. 155-163. Academic Press, London.

Chen, C. M. & Melitz, D. K. (1979) Cytokinin biosynthesis in a cell-free system from cytokinin-autotrophic tobacco tissue cultures. *FEBS Letters* **107:** 15-20.

Cheplick, G. P. (1997) Effects of endophytic fungi on the phenotypic plasticity of *Lolium perenne* (Poaceae). *American Journal of Botany* **84:** 34-40.

Christensen, M. J., Latch, G. C. M. & Tapper, B. A. (1991) Variation within isolates of *Acremonium* endophytes from perennial rye-grasses. *Mycological Research* **95:** 918-923.

Christensen, M. J., Leuchtmann, A., Rowan, D. D. & Tapper, B. A. (1993) Taxonomy of *Acremonium* endophytes of tall fescue (*Festuca arundinacea),* meadow fescue (*F. pratensis)* and perennial ryegrass (*Lolium perenne*). *Mycological Research* **97:** 1083-1092.

Clay, K. (1987) Effects of fungal endophytes on the seed and seedling biology of *Lolium perenne* and *Festuca arundinacea. Oecologia* **73:** 358-362.

Clay, K., Marks, S. & Cheplick, G. P. (1993) Effects of insect herbivory and fungal endophyte infection on competitive interactions among grasses. *Ecology* **74:** 1767-1777.

Clement, S. L., Pike, K. S., Kaiser, W. J. & Wilson, A. D. (1991) Resistance of endophyte-infected plants of tall fescue and perennial ryegrass to the Russian wheat aphid (Homoptera: Aphididae). *J.Kans.Entomol.Soc.* **63:** 646-648.

Colbere-Garapin, F., Horodniceanu, F., Kourilsky, P. & Garapin, A. C. (1981) A new dominant hybrid selective marker for higher eukaryotic cells. *Journal of Molecular Biology* **150:** 1-14.

Crespi, M., Messens, E., Caplan, A. B., Montagu, M. v., Desomer, J. & Van Montagu, M. (1992) Fasciation induction by the phytopathogen *Rhodococcus fascians* depends upon a linear plasmid encoding a cytokinin synthase gene.

*EMBO Journal* **11:** 795-804.

Edwards, K., Johnstone, C. & Thompson, C. (1991) A simple and rapid method for the preparation of plant genomic DNA for PCR analysis. *Nucleic Acids Res.* **19:** 1349.

Faiss, M., Zalubilova, J., Strnad, M. & Schmulling, T. (1997) Conditional transgenic expression of the ipt gene indicates a function for cytokinins in paracrine signaling in whole tobacco plants. *Plant Journal* **12:** 401-415.

Funk, C. R., Halisky, P. M., Johnson, M. C., Siegel, M. R., Stewart, A. V., Ahmad, S., Hurley, R. H. & Harvey, I. C. (1983) An endophytic fungus and resistance to sod webworms: association in *Lolium perenne* L. *Biotechnology* **1:** 189-191.

Garnier, F., Carpin, S., Label, P., Creche, J., Rideau, M. & Hamdi, S. (1996) Effect of cytokinin on alkaloid accumulation in periwinkle callus cultures transformed with a light-inducible *ipt* gene. *Plant Science* **120:** 47-55.

Herd, S., Christensen, M. J., Saunders, K., Scott, B. B. & Schmid, J. (1997) Quantitative assessment of *in Planta* distribution of metabolic activity and gene expression of Neotyphodium endophytes.: pp. 57-60. *Neotyphodium/grass interactions.* New York (USA). Plenum Press.

Hobbie, L., Timpte, C. & Estelle, M. (1994) Molecular genetics of auxin and cytokinin. *Plant Mol Biol.* **26:** 1499-1519.

Jones, R. J. & Schreiber, B. M. N. (1997) Role and function of cytokinin oxidase in plants. *Plant Growth Regulation* **23:** 123-134.

Latch, G. C. M. (1997) An overview of Neotyphodium-grass interactions.: pp. 1-11. *Neotyphodium/grass interactions.* New York (USA). Plenum Press.

Latch, G. C. M. & Christensen, M. J. (1985) Artificial infection of grasses with endophytes. *Annals of Applied Biology* **107:** 17-24.

Latch, G. C. M., Christensen, M. J. & Samuels, G. J. (1984) Five endophytes of *Lolium* and *Festuca* in New Zealand. *Mycotaxon* **20:** 535-550.

Latch, G. C. M., Hunt, W. F. & Musgrave, D. R. (1985) Endophytic fungi affect growth of perennial ryegrass. *New Zealand Journal of Agricultural Research* **28:** 165-168.

Latch, G. C. M., Potter, L. R. & Tyler, B. F. (1987) Incidence of endophytes in seeds from collections of *Lolium* and *Festuca* species. *Annals of Applied Biology* **111:** 59-64.

Letham, D. S. & Palni, L. M. S. (1983) The biosynthesis and metabolism of cytokinin. *Annual Review of Plant Physiology* **34**: 163-197.

Li, Y., Hagen, G. & Guilfoyle, T. J. (1992) Altered morphology in transgenic tobacco plants that overproduce cytokinins in specific tissues and organs. *Developmental Biology* **153** : 386-395.

Martineau, B., Summerfelt, K. R., Adams, D. F. & DeVerna, J. W. (1995) Production of high solids tomatoes through molecular modification of levels of the plant growth regulator cytokinin. *Bio Technology* **13:** 250-254.

McKenzie, M. J., Mett, V., Reynolds, P. H. S. & Jameson, P. E. (1998) Controlled cytokinin production in transgenic tobacco using a copper-inducible promoter. *Plant Physiology* **116:** 969-977.

Medford, J. I., Horgan, R., El-Sawi, Z. & Klee, H. J. (1989) Alterations of endogenous cytokinins in transgenic plants using a chimeric isopentenyl transferase gene. *Plant Cell* **1**: 403-413.

Miller, C. O., Skoog, F., Okomura, F. S., vonSaltza, M. H. & Strong, F. M. (1956) Isolation, structure and synthesis of kinetin, a substance promoting cell division. *Journal of the American Chemical Society* **78:** 1345-1350.

Mok, M. C. (1994) Cytokinins and plant development - An overview. In *Cytokinins:* chemistry, *activity, and function* (D. W. Mok & M. C. Mok, eds): pp. 155-166. CRC Press, Inc., Boca Raton.

Moon, C. D., Tapper, B. A. & Scott, B. (1999) Identification of *Epichloë* endophytes *in planta* by a microsatellite-based PCR fingerprinting assay with automated analysis. *Applied and Environmental Microbiology* **65:** 1268-1279.

Murray, F. R., Latch, G. C. M. & Scott, D. B. (1992) Surrogate transformation of perennial ryegrass, *Lolium perenne,* using genetically modified *Acremonium* endophyte. *Molecular and General Genetics* **233:** 1-9.

Oliver, R. P., Roberts, I. N., Harling, R., Kenyon, L., Punt, P. J., Dingemanse, M. A. & van den Hondel, C. A. M. J. J. (1987) Transformation of *Fulvia Fulva,* a fungal pathogen of tomato, to hygromycin B resistance. *Current Genetics* **12:** 231-233.

Punt, P. J., Oliver, R., Dingemanse, M. A., Pouwels, P. H. & van den Hondel, C. A. M. J. J. (1987) Transformation of *Aspergillus* based on the hygromycin B resistance marker from *Escherichia coli. Gene* **56:** 117-124.

Reinecke, D. M., Brenner, M. L. & Rubenstein, I. (1992) Cytokinin biosynthesis in developing *Zea mays* kernels. *Plant Physiology* **99 (Suppl).**

Roberts, I. N., Oliver, R. P., Punt, P. J. & van den Hondel, C. A. M. J. J. (1989) Expression of the *Escherichia coli* □-glucuronidase gene in industrial and phytopathogenic filamentous fungi. *Current Genetics* **15:** 177-180.

Roeckel, P., Oancia, T. & Drevet, J. R. (1998) Phenotypic alterations and component analysis of seed yield in transgenic *Brassica napus* plants expressing the *tzs* gene. *Physiologia Plantarum* **102:** 243-249.

Rowan, D. D. & Gaynor, D. L. (1986) Isolation of feeding deterrents against Argentine stem weevil from ryegrass infected with the endophyte *Acremonium loliae. Journal of Chemical Ecology* **12:** 647-658.

Sambrook,J., Fritsch,E.F., & Maniatis,T. (1987) *Molecular cloning: A laboratory manual.,* 2nd ed. edn, Cold Springer Harbor Press, Cold Spring Harbor, N.Y..

Shaw, S. L., Yeh, E., Bloom, K. & Salmon, E. D. (1997) Imaging green fluorescent protein fusion proteins in *Saccharomyces cerevisiae. Curr.Biol.* **7**: 701-704.

Sivan, A., Stasz, T. E., Hemmat, M., Hayes, C. K. & Harman, G. E. (1992) Transformation of *Trichoderma* spp. with plasmids conferring hygromycin B resistance. *Mycologia* **84:** 687-694.

Smart, C., Scofield, S., Bevan, M. & Dyer, T. (1991) Delayed leaf senescence in tobacco plants transformed with *tmr,* a gene for cytokinin production in *Agrobacterium. Plant Cell* **3**: 647-656.

Smigocki, A. C. (1991) Cytokinin content and tissue distribution in plants transformed by a reconstructed isopentenyl transferase gene. *Plant Molecular Biology* **16**: 105-115.

Smigocki, A. C. (1995) Expression of a wound-inducible cytokinin biosynthesis gene in transgenic tobacco: correlation of root expression with induction of cytokinin effects. *Plant Science* **109:** 153-163.

Southern, E. M. (1975) Detection of specific sequences among fragments separated by gel electrophoresis. *Journal of Molecular Biology* **98:** 503-517.

Tsai, H. F., Siegel, M. R. & Schardl, C. L. (1992) Transformation of *Acremonium coenophialum,* a protective fungal symbiont of the grass *Festuca arundinacea. Current Genetics* **22:** 399-406.

Ulvskov, P., Nielsen, T. H., Seiden, P. & Marcussen, J. (1992) Cytokinins and leaf development in sweet pepper *(Capsicum annuum* L.). *Planta* **188:** 70-77.

Yunus, A., Kawamata, S., Shimanuki, T., Murakami, Y., Ichinose, Y., Shiraishi, T. & Yamada, T. (1999) Transformation of the endophyte *Neotyphodium* with the iaaM gene. *Annals of the Phytopathological Society of Japan* **65:** 192-196.

## LEGENDS TO FIGURES

### Figure 1

**A.** The start codon region of the *tzs* sequence (Beaty.J.S. *et al.*, 1986) from pNEO/tzs and the two designed forward primers for amplification of the *tzs* gene. The nucleotide and amino acid sequence of the two constructs are compared to the original *tzs* gene. **B.** The new *tzs* plasmids pLM18.tzs and pLM19.tzs are similar except the sequence differences indicated between the arrows (lower case). Start codon is bold and the *Nco*I restriction site is underlined. The *tzs* gene is under the control of the *gpd* promoter and the *trpC* terminator both from *Aspergillus nidulans.*

### Figure 2

Southern blot hybridization of genomic DNA from *tzs* transformants before inoculation into plants (a) and after re-isolation from plants (b). **A.** *Bgl*II digested genomic DNA. Both tzs plasmids has a single *Bgl*II restriction site. **B.** *Cla*I digested genomic DNA. The tzs plasmids has no *Cla*I restriction site. Lane M, *Hin*dIII digested Lambda DNA marker, sizes in Kb. Hybridization probe was the 486bp *tzs* gene sequence *Eco*RI/*Sal*I digestion of the pNEO/tzs plasmid.

### Figure 3

ELISA analysis of 18 days old culture filtrate of tzs transformants (a) before inoculation and (b) after re-isolation from host plants. **A.** Cytokinin concentration of the culture filtrate. **B.** The cytokinin production compared to the amount of mycelium. Zeatin and [9R]iP (= isopentenyl adenosine) were used as standards in the immunoassays. Values are average from two replicate flasks.

### Figure 4

Southern hybridization of *Bgl*I digested genomic DNA. The DNA was extracted from the mycelium of the cultures (shown in figure 3) of the transformants before inoculation into plants (a) and after re-isolation from plants (b). **A.** The 0.7 % agarose gel is shown since varying amount of DNA was extracted from the limited amount of mycelium from the liquid culture (fig. 3). **B.** Southern blot hybridization. Hybridization probe was the 486 bp *Eco*RI/*Sal*I fragment of the pNEO/tzs plasmid containing the *tzs* gene sequence. Lane M, *Hin*dIII digested Lambda DNA marker, size in Kb.

### Figure 5

Cytokinins from culture filtrates of tzs transformants separated by reverse phase HPLC. Immunoreactive material in the collected fractions was quantified by ELISA. **A.** G31 antiserum specific for the zeatin group of cytokinins. **B.** G26 antiserum specific for the iP group of cytokinins. The retention time peaks of the cytokinin metabolites are indicated on top of each graph. Z = zeatin; [9G]Z = zeatin N9-glucosides; [9R]Z = zeatin riboside; iP = isopentenyl adenine; [9R]iP = isopentenyl adenosine. Abbreviations of cytokinins follow the convention established (Letham & Palni, 1983). Bars = standard deviation.

**Figure 6**

**A.** The total number of shoots per plant was observed on plants infected with the transformant T19.4 (tzs+) and the wild type isolate LM41-4. Statistical analysis showed a significant difference between the two groups at 5 % level. **B.** Same as figure 5A yet plants infected with the wild type LM37-5 were included in the control group. Statistical analysis showed a significant difference between the two groups at 5 % level.

**Claims**

1.  A method to change the morphological characters of a grass by introducing a gene into a fungal endophyte and infecting said grass with said endophyte.

2.  A method according to claim 1 wherein said grass belongs to any of the genera Lolium, Festuca, Poa, Dactylis, Agrostis and Phleum.

3.  A method according to claim 1 wherein said endophyte belongs to the genus Neotyphodium.

4.  A method according to claim 1 wherein said gene encodes a plant growth regulator.

5.  A method according to claim 4 wherein said plant growth regulator is a cytokinin.

6.  A method according to claim 1 wherein said morphological characters is either tillering, stem production, onset of senescens, root morphology or seed producction.

7.  A method according to claim 1 wherein

        1) said grass is Lolium perenne
        2) said endophyte is Neotyphodium lolii
        3) said gene is tzs from Agrobacterium tumefaciens

8.  A plant obtained according to a method described in any of claims 1-7.

9.  The progeny of the plant according to claim 8, said progeny being in the form of a seed or otherwise generatively or vegetatively propagated.

# Figure 1

**A**

The designed forward primers and the *tzs* template:

pLM18.tzs:  5'-gg cat <u>gcc atg g</u>ta tta ctc cat ctc atc tac g-3'
                     M V L L H L I Y

pLM19.tzs:  5'-gg cat <u>gcc atg g</u>ta --- ctc cat ctc atc tac g-3'
                     M V    L H L I Y

pNEO/tzs:   5'-ag gcg cac atg --- tta ctc cat ctc atc tac g-3'
                     M    L L H L I Y

**B**

Noo I
2293 GACATCA**CCAT**G t a TT ACT CCAT    **pLM18.tzs** (5464bp)

Noo I
2293 GACATCA**CCAT**G qT ACT CCAT    **pLM19.tzs** (5461bp)

AT GTT ACT C CAT    ***tzs* sequence**

# Figure 2

# Figure 3

# Figure 4

# Figure 5

Figure 6

A  *Lolium perenne*
var. Tivoli

B

**European Patent Office**

## EUROPEAN SEARCH REPORT

Application Number

EP 00 61 0096

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | MURRAY F R ET AL: "SURROGATE TRANSFORMATION OF PERENNIAL RYEGRASS LOLIUM-PERENNE USING GENETICALLY MODIFIED ACREMONIUM ENDOPHYTE" MOLECULAR & GENERAL GENETICS, vol. 233, no. 1-2, 1992, pages 1-9, XP002161905 ISSN: 0026-8925 * the whole document * | 1-9 | C12N15/82 C12N9/10 C12N15/31 A01H1/00 |
| D,Y | YUNUS AHMAD ET AL: "Transformation of the endophyte Neotyphodium with the iaaM gene." ANNALS OF THE PHYTOPATHOLOGICAL SOCIETY OF JAPAN, vol. 65, no. 2, April 1999 (1999-04), pages 192-196, XP000982677 ISSN: 0031-9473 * the whole document * | 1-9 | |
| Y | WO 98 10734 A (FANG YIWEN ;HIRSCH ANN M (US); UNIV CALIFORNIA (US)) 19 March 1998 (1998-03-19) * the whole document * | 7 | TECHNICAL FIELDS SEARCHED (Int.Cl.7) C12N |

The present search report has been drawn up for all claims

| Place of search | Date of completion of the search | Examiner |
|---|---|---|
| THE HAGUE | 2 March 2001 | Oderwald, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 00 61 0096

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2001

| Patent document cited in search report | Publication date | Patent family member(s) | Publication date |
|---|---|---|---|
| WO 9810734 A | 19-03-1998 | AU 4341997 A | 02-04-1998 |

EPO FORM P0459

For more details about this annex : see Official Journal of the European Patent Office, No. 12/82